(19) Europäisches Patentamt
European Patent Office
Office européen des brevets

(11) EP 4 707 263 A1

(12) **EUROPEAN PATENT APPLICATION**
published in accordance with Art. 153(4) EPC

(43) Date of publication:
**11.03.2026 Bulletin 2026/11**

(21) Application number: **25761925.4**

(22) Date of filing: **26.02.2025**

(51) International Patent Classification (IPC):
***C07B 39/00*** (2006.01) ***C07C 309/81*** (2006.01)
***B02C 17/14*** (2006.01) ***C07C 309/85*** (2006.01)
***B02C 19/20*** (2006.01) ***C07C 309/86*** (2006.01)
***C01D 3/02*** (2006.01) ***C07C 309/87*** (2006.01)
***C07C 17/20*** (2006.01) ***C07C 309/88*** (2006.01)

(52) Cooperative Patent Classification (CPC):
**B02C 17/14; B02C 19/20; C01D 3/02; C07B 39/00; C07C 17/20; C07C 22/08; C07C 45/63; C07C 49/213; C07C 49/217; C07C 49/84; C07C 51/363; C07C 51/58; C07C 57/72; C07C 63/04; C07C 63/70;** (Cont.)

(86) International application number:
**PCT/JP2025/006741**

(87) International publication number:
**WO 2025/183030 (04.09.2025 Gazette 2025/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.02.2024 JP 2024026766**
**11.04.2024 JP 2024064110**

(71) Applicants:
- **Daikin Industries, Ltd.**
  **Osaka-shi, Osaka 530-0001 (JP)**
- **Nagoya Institute Of Technology**
  **Nagoya-shi, Aichi 466-8555 (JP)**

(72) Inventors:
- **MUKAE, Hirofumi**
  **Osaka-Shi, Osaka 530-0001 (JP)**
- **KISHIKAWA, Yosuke**
  **Osaka-Shi, Osaka 530-0001 (JP)**
- **HOSHIYA, Naoyuki**
  **Osaka-Shi, Osaka 530-0001 (JP)**
- **HOSOKAWA, Moe**
  **Osaka-Shi, Osaka 530-0001 (JP)**
- **HIGASHI, Masahiro**
  **Osaka-Shi, Osaka 530-0001 (JP)**
- **NAKANISHI, Kanako**
  **Osaka-Shi, Osaka 530-0001 (JP)**
- **SHIBATA, Norio**
  **Nagoya-shi, Aichi 466-8555 (JP)**
- **HATTORI, Masashi**
  **Nagoya-shi, Aichi 466-8555 (JP)**

(74) Representative: **Hoffmann Eitle**
**Patent- und Rechtsanwälte PartmbB**
**Arabellastraße 30**
**81925 München (DE)**

(54) **FLUORINATING AGENT AND FLUORINATION METHOD**

(57) The disclosure provides a fluorinating agent that can be produced by a simple method, and a fluorination method including using a fluorinating agent that can be produced by a simple method. The disclosure relates to a fluorinating agent containing a fluorine-containing compound and a fluoride ion.

(52) Cooperative Patent Classification (CPC): (Cont.)
**C07C 65/21; C07C 201/12; C07C 205/11; C07C 303/02; C07C 303/22; C07C 309/80; C07C 309/81; C07C 309/85; C07C 309/86; C07C 309/87; C07C 309/88; C07D 207/08; C07D 213/62; C07D 233/84; C07D 333/34; C08F 8/26; C08J 3/12; C08K 3/22; C08K 5/05; C08K 5/09; C08K 5/095; C08K 5/42; C08L 27/12; C08L 27/16; C08L 27/18**

## Description

TECHNICAL FIELD

The disclosure relates to fluorinating agents and fluorination methods.

BACKGROUND ART

Potassium fluoride (KF) is a known fluorinating agent for organochlorides. Potassium fluoride is commonly synthesized by the reaction of HF and KOH and is processed into fine powder by spray drying (e.g., see Non-Patent Literature 1).

CITATION LIST

Non-Patent Literature

Non-Patent Literature 1: "2019 Nendo-ban 17019-no Kagaku Shohin (17019 Chemical Products, FY2019)", The Chemical Daily Co., Ltd., 2019, p. 187

SUMMARY OF INVENTION

- Technical Problem

A traditional process of producing potassium fluoride, which is known as a fluorinating agent, involves using a strong acid and a strong base and spray-drying the product into fine powder, and is therefore complicated.

The disclosure aims to provide a fluorinating agent that can be produced by a simple method and a fluorination method including using a fluorinating agent that can be produced by a simple method.

- Solution to Problem

The disclosure (1) relates to a fluorinating agent containing a fluorine-containing compound and a fluoride ion.

The disclosure (2) relates to the fluorinating agent according to the disclosure (1), wherein the fluorinating agent is solid at 25°C.

The disclosure (3) relates to the fluorinating agent according to the disclosure (1) or (2), wherein a counterion of the fluoride ion includes at least one selected from the group consisting of an alkali metal, an alkaline earth metal, and $NR^1_4$ (wherein $R^1$s are the same as or different from each other and are each H or a C1-C10 organic group).

The disclosure (4) relates to the fluorinating agent according to any one of the disclosures (1) to (3), further containing a base.

The disclosure (5) relates to the fluorinating agent according to the disclosure (4), wherein the base is solid at 25°C.

The disclosure (6) relates to the fluorinating agent according to the disclosure (4) or (5), wherein the base has a pKa of 8 to 40.

The disclosure (7) relates to the fluorinating agent according to any one of the disclosures (4) to (6), wherein the base includes at least one selected from the group consisting of a compound represented by $R^{10}OM$ (wherein $R^{10}$ is H or a C1-C10 organic group; M is a metal or $NR^1_4$ (wherein $R^1$s are the same as or different from each other and are each H or a C1-C10 organic group)) and a metal carbonate.

The disclosure (8) relates to the fluorinating agent according to any one of the disclosures (4) to (7), wherein the base includes at least one selected from the group consisting of sodium hydroxide, potassium hydroxide, cesium hydroxide, cesium carbonate, sodium methoxide, potassium methoxide, sodium ethoxide, potassium ethoxide, sodium t-butoxide, and potassium t-butoxide.

The disclosure (9) relates to the fluorinating agent according to any one of the disclosures (4) to (8), wherein the base is contained in an amount of 0.1 to 70% by mass.

The disclosure (10) relates to the fluorinating agent according to any one of the disclosures (1) to (9), wherein the fluorine-containing compound is solid at 25°C.

The disclosure (11) relates to the fluorinating agent according to any one of the disclosures (1) to (10), wherein the fluorine-containing compound is a fluorine-containing polymer.

The disclosure (12) relates to the fluorinating agent according to any one of the disclosures (1) to (11), wherein the fluorine-containing compound is a fluorine-containing polymer containing a polymerized unit based on at least one

monomer selected from the group consisting of tetrafluoroethylene, difluoroethylene, chlorotrifluoroethylene, hexafluoropropylene, perfluoro(alkyl vinyl ether), trifluoroethylene, and monofluoroethylene.

The disclosure (13) relates to the fluorinating agent according to any one of the disclosures (1) to (12), wherein the fluorine-containing compound includes at least one selected from the group consisting of polytetrafluoroethylene, polyvinylidene fluoride, and polychlorotrifluoroethylene.

The disclosure (14) relates to a fluorinating agent which is a composition obtainable by subjecting a fluorine-containing compound and a base to mechanochemical treatment.

The disclosure (15) relates to a fluorination method comprising fluorinating a target using the fluorinating agent according to the disclosure (14).

The disclosure (16) relates to a fluorination method comprising fluorinating a target using a fluorinating agent that comprises a fluorine-containing compound and a fluoride ion.

The disclosure (17) relates to the fluorination method according to the disclosure (15) or (16), wherein the fluorinating agent is solid at 25°C.

The disclosure (18) relates to the fluorination method according to the disclosure (16) or (17), wherein the fluorine-containing compound is solid at 25°C.

The disclosure (19) relates to the fluorination method according to any one of the disclosures (16) to (18), wherein the fluorine-containing compound is a fluorine-containing polymer.

The disclosure (20) relates to the fluorination method according to any one of the disclosures (16) to (19), wherein the fluorine-containing compound is a fluorine-containing polymer containing a polymerized unit based on at least one monomer selected from the group consisting of tetrafluoroethylene, difluoroethylene, chlorotrifluoroethylene, hexafluoropropylene, perfluoro(alkyl vinyl ether), trifluoroethylene, and monofluoroethylene.

The disclosure (21) relates to the fluorination method according to any one of the disclosures (16) to (20), wherein the fluorine-containing compound includes at least one selected from the group consisting of polytetrafluoroethylene, polyvinylidene fluoride, and polychlorotrifluoroethylene.

The disclosure (22) relates to the fluorination method according to any one of the disclosures (16) to (21), wherein a counterion of the fluoride ion includes at least one selected from the group consisting of an alkali metal, an alkaline earth metal, and $NR^1_4$ (wherein $R^1$s are the same as or different from each other and are each H or a C1-C10 organic group).

The disclosure (23) relates to the fluorination method according to any one of the disclosures (15) to (22), wherein the fluorinating agent further contains a base.

The disclosure (24) relates to the fluorination method according to the disclosure (23), wherein the base is solid at 25°C.

The disclosure (25) relates to the fluorination method according to the disclosure (23) or (24), wherein the base includes at least one selected from the group consisting of a compound represented by $R^{10}OM$ (wherein $R^{10}$ is H or a C1-C10 organic group; M is a metal or $NR^1_4$ (wherein $R^1$s are the same as or different from each other and are each H or a C1-C10 organic group)) and a metal carbonate.

The disclosure (26) relates to the fluorination method according to any one of the disclosures (23) to (25), wherein the base includes at least one selected from the group consisting of sodium hydroxide, potassium hydroxide, cesium hydroxide, cesium carbonate, sodium methoxide, potassium methoxide, sodium ethoxide, potassium ethoxide, sodium t-butoxide, and potassium t-butoxide.

The disclosure (27) relates to the fluorination method according to any one of the disclosures (23) to (26), wherein the base is contained in an amount of 0.1 to 70% by mass relative to the fluorinating agent.

The disclosure (28) relates to the fluorination method according to any one of the disclosures (16) to (27), wherein the fluorinating agent is obtainable by subjecting a fluorine-containing compound and a base to mechanochemical treatment.

The disclosure (29) relates to the fluorination method according to any one of the disclosures (15) to (28), further including purifying a crude product obtained by the fluorination and collecting the target fluorinated.

The disclosure (30) relates to the fluorination method according to any one of the disclosures (15) to (29), wherein the fluorination is performed under dry conditions.

The disclosure (31) relates to the fluorination method according to any one of the disclosures (15) to (30), wherein the fluorination is performed by subjecting the fluorinating agent and the target to mechanochemical treatment.

The disclosure (32) relates to the fluorination method according to any one of the disclosures (15) to (31), wherein the production of the fluorinating agent and the fluorination are performed continuously.

The disclosure (33) relates to the fluorination method according to any one of the disclosures (15) to (32), wherein the fluorinating agent is used in an amount of 1.0 to 3.0 equivalents relative to 1 equivalent of a group that is potentially replaced by a fluorine atom in the target.

The disclosure (34) relates to the fluorination method according to any one of the disclosures (15) to (33), wherein the target is an organic compound containing at least one selected from the group consisting of a chlorine atom and a bromine atom.

- Advantageous Effects of Invention

The disclosure can provide a fluorinating agent that can be produced by a simple method and a fluorination method including using a fluorinating agent that can be produced by a simple method.

DESCRIPTION OF EMBODIMENTS

The disclosure is described in detail below.

The disclosure relates to a fluorinating agent containing a fluorine-containing compound and a fluoride ion (hereinafter, also referred to as the fluorinating agent (1) of the disclosure).

The fluorinating agent (1) of the disclosure has the aforementioned structure and therefore can be produced by a simple method. The presence of a fluorine-containing compound, which absorbs less water, around a component containing a fluoride ion can reduce adhesion of this component even when water is absorbed and allows the powdery form to be maintained. Thus, the fluorinating agent (1) of the disclosure has excellent handleability even when left in the air.

The fluorine-containing compound in the fluorinating agent (1) of the disclosure is any compound having a fluorine atom, and may be a compound having a fluorine atom bonded to a carbon atom, preferably an organic compound having a fluorine atom bonded to a carbon atom.

In order to achieve much better handleability, the fluorine-containing compound is preferably solid at 25°C.

The fluorine-containing compound is preferably a fluorine-containing high-molecular-weight compound, more preferably a fluorine-containing polymer.

The fluorine-containing polymer preferably contains a polymerized unit based on at least one monomer selected from the group consisting of tetrafluoroethylene (TFE), difluoroethylene, chlorotrifluoroethylene (CTFE), hexafluoropropylene (HFP), perfluoro(alkyl vinyl ether) (PAVE), trifluoroethylene, and monofluoroethylene.

Examples of the difluoroethylene include vinylidene fluoride (VdF) and 1,2-difluoroethylene.

The fluorine-containing polymer more preferably contains a polymerized unit based on at least one monomer selected from the group consisting of TFE, difluoroethylene, and CTFE, still more preferably contains a polymerized unit based on at least one monomer selected from the group consisting of TFE, VdF, and CTFE, further preferably contains a polymerized unit based on at least one monomer selected from the group consisting of TFE and VdF, particularly preferably contains a polymerized unit based on VdF.

The fluorine-containing polymer may be a fluororesin or may be a fluoroelastomer.

Examples of the fluororesin include polytetrafluoroethylene (PTFE), a tetrafluoroethylene (TFE)/perfluoro(alkyl vinyl ether) (PAVE) copolymer (PFA), a TFE/hexafluoropropylene (HFP) copolymer (FEP), an ethylene (Et)/TFE copolymer (ETFE), an Et/TFE/HFP copolymer (EFEP), polychlorotrifluoroethylene (PCTFE), a chlorotrifluoroethylene (CTFE)/TFE copolymer, a CTFE/TFE/PAVE copolymer, an Et/CTFE copolymer, polyvinyl fluoride (PVF), polyvinylidene fluoride (PVdF), a vinylidene fluoride (VdF)/TFE copolymer, a VdF/HFP copolymer, a VdF/TFE/HFP copolymer, a VdF/HFP/(meth)acrylic acid copolymer, a VdF/CTFE copolymer, a VdF/pentafluoropropylene copolymer, a VdF/PAVE/TFE copolymer, and a TFE/perfluoroalkyl allyl ether copolymer. These may be used alone or in any combination. The perfluoroalkyl allyl ether is a monomer represented by $CF_2=CFCF_2-O-Rf^1$ (wherein $Rf^1$ is a C1-C5 perfluoroalkyl group).

Examples of the fluoroelastomer include a vinylidene fluoride-based (VdF-based) fluoroelastomer, a tetrafluoroethylene (TFE)/propylene (Pr)-based fluoroelastomer, a TFE/Pr/VdF-based fluoroelastomer, an ethylene (Et)/hexafluoropropylene (HFP)-based fluoroelastomer, an Et/HFP/VdF-based fluoroelastomer, an Et/HFP/TFE-based fluoroelastomer, a fluorosilicone-based fluoroelastomer, and a fluorophosphazene-based fluoroelastomer. These may be used alone or in any combination.

Examples of the VdF-based fluoroelastomer include a VdF/HFP copolymer, a VdF/TFE/HFP copolymer, a VdF/chlorotrifluoroethylene (CTFE) copolymer, a VdF/CTFE/TFE copolymer, a VdF/perfluoro(alkyl vinyl ether) (PAVE) copolymer, a VdF/TFE/PAVE copolymer, a VdF/HFP/PAVE copolymer, a VdF/HFP/TFE/PAVE copolymer, a VdF/TFE/Pr copolymer, a VdF/Et/HFP copolymer, and a copolymer of VdF and a fluorine-containing monomer represented by the following formula (1). Formula (1):

$$CH_2=CFRf^2 \qquad (1)$$

wherein $Rf^2$ is a C1-C12 linear or branched fluoroalkyl group.

The fluorine-containing polymer preferably includes at least one selected from the group consisting of a fluororesin and a fluoroelastomer, more preferably includes at least one selected from the group consisting of PTFE, FEP, PFA, PVdF, PCTFE, and a fluoroelastomer, still more preferably includes at least one selected from the group consisting of PTFE, PVdF, and PCTFE, further preferably includes at least one selected from the group consisting of PTFE and PVdF, and is particularly preferably PVdF.

The fluorine-containing polymer is also preferably a perhaloresin, more preferably includes at least one selected

from the group consisting of a perfluororesin and PCTFE, still more preferably includes at least one selected from the group consisting of PTFE, PFA, FEP, and PCTFE, further preferably at least one selected from the group consisting of PTFE, PFA, and FEP.

The fluorine-containing polymer also preferably includes at least one selected from the group consisting of a perfluororesin and polydifluoroethylene, and more preferably includes at least one selected from the group consisting of PTFE and polydifluoroethylene.

The fluorine-containing compound is not necessarily a polymer as long as it is solid at 25°C, and may be a fluorine-containing low-molecular-weight compound. The fluorine-containing low-molecular-weight compound may be at least one fluorine-containing organic acid compound selected from the group consisting of a compound (I) represented by the following formula (I):

$$Y\text{-}(CF_2)_{x1}\text{-}(CH2)_{y1}\text{-}A \qquad (I)$$

(wherein Y is H or F; x1 is an integer of 4 or greater; y1 is an integer of 0 to 3; and A is $-SO_3M^I$ or $-COOM^I$, where $M^I$ is H, $NH_4$, Li, Na, Mg, Al, K, or Ca) and a compound (II) represented by the following formula (II):

$$F\text{-}(CF_2)_{x2}O(CFXCF_2O)_{y2}\text{-}CFX\text{-}A \qquad (II)$$

(wherein x2 is an integer of 1 or greater; y2 is an integer of 0 to 10; X is F or $CF_3$; and A is $-SO_3M^{II}$ or $-COOM^{II}$, where $M^{II}$ is H, $NH_4$, Li, Na, Mg, Al, K, or Ca).

Examples of the compound (I) include a fluorocarboxylic acid and a salt thereof, preferably a perfluorocarboxylic acid and a salt thereof, such as perfluorooctanoic acid and a salt thereof (which are also collectively referred to as "PFOA"). Examples of the salts include ammonium salts and sodium salts, with ammonium salts such as ammonium perfluorooctanoate (especially also referred to as "APFO") being preferred.

Examples of the compound (I) also include fluorosulfonic acid and a salt thereof, preferably perfluorosulfonic acid and a salt thereof, such as perfluorooactanesulfonic acid and a salt thereof (which are also collectively referred to as "PFOS"). Examples of the salts include ammonium salts and sodium salts.

Examples of the compound (II) include perfluoroether carboxylic acid and a salt thereof, such as 2,3,3,3-tetrafluoro-2-[1,1,2,3,3,3-hexafluoro-2-(trifluoromethoxy)propoxy]-propanoic acid.

The fluorine-containing low-molecular-weight compound may be adsorbed on an adsorbent. In this embodiment, the fluorinating agent (1) of the disclosure may contain a solid composed of the fluorine-containing low-molecular-weight compound adsorbed on an adsorbent.

The adsorbent may be any solid that can adsorb the fluorine-containing low-molecular-weight compound, and is preferably at least one selected from the group consisting of activated carbon, silica gel, clay, a metal organic framework (MOF), and zeolite.

The fluorine-containing polymer may have been heated to the melting point or higher once, and may be a molded one. The fluorine-containing polymer may be a pulverized one after molding. In terms of reactivity in the production method to be described later, the particle size is preferably small.

The amount of the fluorine-containing compound contained relative to the fluorinating agent (1) of the disclosure is preferably 0.1% by mass or more, more preferably 0.3% by mass or more, still more preferably 0.5% by mass or more, while preferably 90% by mass or less, more preferably 85% by mass or less, still more preferably 80% by mass or less, yet still more preferably 50% by mass or less, further preferably 30% by mass or less, further more preferably 10% by mass or less, still further more preferably 5% by mass or less, yet still further more preferably 3% by mass or less, particularly preferably 1% by mass or less.

The amount of the fluorine-containing compound contained is preferably small.

Preferably, the fluorinating agent (1) of the disclosure is substantially free from a fluorine-containing organic compound. The phrase "substantially free from a fluorine-containing organic compound" means that the amount of organofluorine contained relative to the fluorinating agent (1) of the disclosure is 10% by mass or less, preferably 5% by mass or less, more preferably 3% by mass or less, still more preferably 2% by mass or less, further preferably 1% by mass or less, particularly preferably 0.1% by mass or less. The lower limit may be, but is not limited to, 0% by mass or may be 0.0001% by mass.

The amount of organofluorine in the fluorinating agent is determined by combustion ion chromatography.

The organofluorine means fluorine bonded to carbon.

The fluorinating agent (1) of the disclosure contains a fluoride ion. The amount of the fluoride ion contained relative to the fluorinating agent is preferably 0.5% by mass or more, more preferably 1.0% by mass or more, still more preferably 3.0% by mass or more, particularly preferably 5.0% by mass or more, while preferably 50% by mass or less, more preferably 30% by mass or less, still more preferably 20% by mass or less, particularly preferably 15% by mass or less, and may be 10% by mass or less.

The fluorinating agent (1) of the disclosure commonly contains not only a fluoride ion but also a counterion. The counterion preferably includes at least one selected from the group consisting of a metal and $NR^1_4$ (wherein $R^1$s are the same as or different from each other and are each H or a C1-C10 organic group). They may be contained in the form of cations.

Examples of the metal include monovalent metals and divalent metals, such as alkali metals (Group 1) and alkaline earth metals (Group 2), specifically Na, K, Li, Cs, and Ca. Preferred among these are alkali metals, more preferred are Na, K, and Cs, and still more preferred is K.

$NR^1_4$ is ammonium (substituted or non-substituted ammonium) and the four $R^1$s in the formula may be the same as or different from each other.

$R^1$ is preferably H or a C1-C10 organic group, more preferably H or a C1-C4 organic group.

The counterion more preferably contains at least one selected from the group consisting of an alkali metal, an alkaline earth metal, and $NR^1_4$ (wherein $R^1$s are the same as or different from each other and are each H or a C1-C10 organic group), still more preferably contains at least one selected from the group consisting of an alkali metal and an alkaline earth metal, further preferably contains an alkali metal, further more preferably contains at least one selected from the group consisting of Na, K, and Cs, and particularly preferably contains K.

The amount of the counterion contained is preferably such that the electric charge thereof balances that of the fluoride ion.

The fluorinating agent (1) of the disclosure may contain a compound having a fluoride ion, or may contain a compound having a fluoride ion and a counterion. Examples of the compound include a metal fluoride and ammonium fluoride; preferred is a metal fluoride, more preferred is an alkali metal fluoride, and still more preferred is potassium fluoride.

The fluorinating agent (1) of the disclosure preferably further contains a base. The fluorinating agent containing not only a fluorine-containing compound but also a base can be produced by subjecting the fluorine-containing compound and the base to mechanochemical treatment, and thus can be produced by a more simple method.

The base is a basic compound, and is preferably a strongly basic compound.

In order to achieve excellent handleability, the base is preferably solid at 25°C.

In order to enable production of a fluorinating agent using a general-purpose device, the base preferably has a pKa (acid dissociation constant at 25°C in water) of preferably 40 or lower, more preferably 35 or lower, still more preferably 20 or lower, while preferably 8 or higher, more preferably 9 or higher, still more preferably 12 or higher, further preferably 15 or higher.

The pKa is determined by neutralization titration.

The base may be either an inorganic base or an organic base, and is preferably an organic base in order to produce a fluorinating agent using a general-purpose device.

The base preferably contains at least one selected from the group consisting of a metal and $NR^1_4$ (wherein $R^1$s are the same as or different from each other and are each H or a C1-C10 organic group). They may be contained in the form of cations.

The metal and $NR^1_4$ are the same as those described above.

The base more preferably contains at least one selected from the group consisting of an alkali metal, an alkaline earth metal, and $NR^1_4$ (wherein $R^1$s are the same as or different from each other and are each H or a C1-C10 organic group), still more preferably contains at least one selected from the group consisting of an alkali metal and an alkaline earth metal, further preferably contains an alkali metal, further more preferably contains at least one selected from the group consisting of Na and K, and particularly preferably contains K.

Examples of the base include a compound represented by $R^{10}OM$ (wherein $R^{10}$ is H or a C1-C10 organic group; M is a metal or $NR^1_4$ (wherein $R^1$s are the same as or different from each other and are each H or a C1-C10 organic group)), a metal carbonate, a metal acetate, a cyclic amine, and a polyamine.

The organic group for $R^{10}$ has a carbon number of preferably 2 or greater, more preferably 3 or greater, while preferably 8 or smaller, more preferably 6 or smaller. $R^{10}$ is preferably H or an alkyl group having a carbon number within the above range, more preferably an alkyl group having a carbon number within the above range, still more preferably a methyl group, an ethyl group, or a t-butyl group.

Examples of the metal and $NR^1_4$ for M include those described above. M is preferably a metal, more preferably an alkali metal or an alkaline earth metal, still more preferably an alkali metal, further preferably Na or K, particularly preferably K.

The metal of the metal carbonate is preferably an alkali metal or an alkaline earth metal, more preferably an alkali metal, still more preferably Na, K, or Cs, particularly preferably Cs.

The metal of the metal acetate is preferably an alkali metal or an alkaline earth metal, more preferably an alkali metal, still more preferably Na or K, particularly preferably K.

Examples of the cyclic amine include 1,8-diazabicyclo[5.4.0]-7-undecene (DBU) and a derivative thereof.

Examples of the polyamine include polyethylene imine.

In order to produce a fluorinating agent by a more simple method, the base preferably includes at least one selected from the group consisting of a compound represented by $R^{10}OM$ and a metal carbonate. In order to facilitate production of a fluorinating agent using a general-purpose device, the base is preferably a compound represented by $R^{10}OM$ where $R^{10}$ is a C1-C10 organic group; more preferably a metal alkoxide; still more preferably an alkali metal alkoxide, further preferably includes at least one selected from the group consisting of an alkali metal methoxide, an alkali metal ethoxide, and an alkali metal t-butoxide, particularly preferably includes at least one selected from the group consisting of sodium methoxide, potassium methoxide, sodium ethoxide, potassium ethoxide, sodium t-butoxide, and potassium t-butoxide.

The base preferably includes at least one selected from the group consisting of an alkali metal hydroxide, an alkali metal carbonate, and an alkali metal alkoxide, more preferably at least one selected from the group consisting of sodium hydroxide, potassium hydroxide, cesium hydroxide, cesium carbonate, sodium methoxide, potassium methoxide, sodium ethoxide, potassium ethoxide, sodium t-butoxide, and potassium t-butoxide.

The amount of the base contained relative to the fluorinating agent (1) of the disclosure is preferably 0.01% by mass or more, more preferably 0.05% by mass or more, still more preferably 0.1% by mass or more, while preferably 90% by mass or less, more preferably 80% by mass or less, still more preferably 70% by mass or less, further preferably 50% by mass or less, further more preferably 30% by mass or less, still further more preferably 20% by mass or less, particularly preferably 10% by mass or less.

The base in an amount within this range can prevent decomposition of the substrate when the fluorinating agent is used for the fluorination, and thus is preferred in terms of yield.

The fluorinating agent (1) of the disclosure preferably contains a compound having a structure in which the fluorine-containing compound is defluorinated. This compound may have a structure formed by defluorinating at least some of the fluorine atoms of the fluorine-containing compound.

In the case where the fluorinating agent (1) of the disclosure contains a base, the compound may be a compound formed by replacing at least some of the fluorine atoms of the fluorine-containing compound with a structure derived from the base, such as a group represented by $R^{10}O$- ($R^{10}$ is defined as described above) (e.g., a hydroxy group or an alkoxy group, preferably an alkoxy group).

The fluorinating agent containing a compound having the above structure can be produced by a more simple method, i.e., decomposition (defluorination) of a fluorine-containing compound.

The presence of a compound having the above structure in the fluorinating agent can be confirmed, for example, by washing the fluorinating agent to remove the components other than the fluorine-containing compound from the fluorinating agent and then analyzing the fluorinating agent by XPS, FT-IR, solid NMR, or the like and performing elemental analysis to measure the carbon and fluorine contents before and after the reaction.

The fluorinating agent (1) of the disclosure may contain a different component to the extent that the effects are not impaired. Examples of the different component include a common filler, a common polymer, and the aforementioned adsorbent.

Examples of the common filler include inorganic fillers such as glass fiber, glass beads, carbon fiber, spherical carbon, carbon black, graphite, silica, alumina, mica, silicon carbide, boron nitride, aluminum nitride, titanium oxide, bismuth oxide, cobalt oxide, magnesium oxide, molybdenum disulfide, bronze, gold, silver, copper, nickel, aluminum fluoride, carbon fluoride, and carbon black.

Examples of the common polymer include polyolefin resins such as polyethylene and polypropylene; polyamide (PA) resins such as nylon 6, nylon 11, nylon 12, nylon 46, nylon 66, nylon 610, nylon 612, and nylon MXD6; polyesters such as polyethylene terephthalate (PET), polybutylene terephthalate (PBT), polyarylate, aromatic polyester (including liquid crystal polyester), and polycarbonate (PC); polyacetal (POM) resin; polyether resins such as polyphenylene oxide (PPO), modified polyphenylene ether, and polyether ether ketone (PEEK); polyamide-imide (PAI) resins such as polyaminobismaleimide; polysulfone resins such as polysulfone (PSF) and polyethersulfone (PES); and vinyl polymers such as ABS resin and poly(4-methylpentene-1) (TPX resin), as well as polyphenylene sulfide (PPS), polyketone sulfide, polyetherimide, polyimide (PI), and epoxy resin. The nylon MXD6 is a crystalline polycondensate obtainable from meta-xylenediamine (MXD) and adipic acid.

The common polymer may be a fluorine-free polymer.

The amount of the different component relative to the fluorinating agent may be 50% by mass or less, and is preferably 30% by mass or less, more preferably 15% by mass or less, still more preferably 10% by mass or less, while the amount may be 0% by mass or more, may be 0.01% by mass or more, may be 0.1% by mass or more, and may be 1% by mass or more.

In order to achieve excellent handleability, the fluorinating agent (1) of the disclosure is preferably solid at 25°C, and is preferably powder.

For the fluorinating agent (1) of the disclosure, the maximum particle size observed with a microscope such as a video microscope is preferably 7 mm or smaller, more preferably 6 mm or smaller, still more preferably 5 mm or smaller, particularly preferably 4 mm or smaller, while preferably 10 μm or greater, more preferably 100 μm or greater, still more

preferably 300 μm or greater, particularly preferably 500 μm or greater.

The fluorinating agent having a maximum particle size within this range does not form a mass and has excellent handleability.

The fluorinating agent (1) of the disclosure can be produced, for example, by mixing a fluorine-containing compound, a compound having a fluoride ion, and optionally a base (hereinafter, also referred to as a production method (1)). The mixing can be performed by any known method without limitation.

In the case of containing a base, the fluorinating agent (1) of the disclosure can be produced by subjecting a fluorine-containing compound and a base to mechanochemical treatment (hereinafter, also referred to as a production method (2)). In order to produce the fluorinating agent (1) in a more simple method, the production method (2) is more preferred. In other words, the fluorinating agent (1) of the disclosure is preferably one obtained by subjecting a fluorine-containing compound and a base to mechanochemical treatment. Also, mechanochemical treatment allows the fluorinating agent to be substantially free from a fluorine-containing organic compound. The fluorinating agent (1) of the disclosure is preferably one that is obtained by subjecting a fluorine-containing compound and a base to mechanochemical treatment and that is substantially free from a fluorine-containing organic compound.

The production method (2) is described in detail below.

In the mechanochemical treatment, the base is used in an amount, relative to 1 equivalent (mole equivalent) of the fluorine-containing compound, of preferably 0.01 equivalents or more, more preferably 0.05 equivalents or more, still more preferably 0.10 equivalents or more, while preferably 10 equivalents or less, more preferably 8 equivalents or less, still more preferably 5 equivalents or less, further preferably 3 equivalents or less, further more preferably 1 equivalent or less, particularly preferably 0.8 equivalents or less.

The production method (2) enables the mechanochemical treatment with a relatively small amount of the base. A small amount of the base can advantageously prevent decomposition of the substrate in the case where the fluorinating agent is used in the fluorination step.

In the case where the fluorine-containing compound is a fluorine-containing polymer, the equivalent is calculated based on the monomers of the fluorine-containing polymer.

The mechanochemical treatment is a treating process of adding any mechanical energy to a reactant (preferably a solid reactant) by a technique such as shearing, compression, expansion, grinding, friction, kneading, mixing, dispersion, disintegration, or shaking to activate the reactant, thereby changing the structure, causing phase transition, or giving reactivity, adsorbability, or catalytic activity, for example.

The mechanochemical treatment may be any mode such as compression shear treatment, impact treatment, or mixing shear friction treatment, with the impact treatment being preferred.

The mechanochemical treatment can be performed by any device that can add mechanical energy by any aforementioned process, such as a known pulverizer or mixer. Examples thereof include pulverizing machines such as a ball mill, a rod mill, a jet mill, a vibrating mill, and a SAG mill; grinding machines such as a rotary quern and a mortar grinder; (horizontal axis rotary) tumbling mixing machines such as a horizontal cylinder mixer, a V-type mixer, a double cone mixer, a cubic mixer, an S-type mixer, and a continuous V-type mixer; (baffle-equipped) tumbling mixing machines such as a horizontal cylinder mixer, a V-type mixer, a double cone mixer, and a ball mill mixer; (rotational oscillating) tumbling mixing machines such as a rocking mixer and a cross-rotary mixer; (horizontal axis rotary) stationary mixing machines such as a ribbon mixer, a paddle mixer, a single shaft rotor mixer, and a pug mill mixer; (vertical axis rotary) stationary mixing machines such as a ribbon mixer, a screw mixer, a planetary mixer, a turbine mixer, a high speed flow mixer, a rotary disc mixer, and a muller mixer; (vibratory) stationary mixing machines such as a vibrating mill and a screen; (fluidized) fluid transmission mixing machines such as a non-uniform fluidized bed mixer, a swirling fluidized bed mixer, a mixer with a rising pipe, and a jet pump mixer; (gravity) fluid transmission mixing machines such as a gravity mixer and a static mixer; and kneading machines such as a double-shaft kneader, a single-shaft kneader, a mixer, and a roll mill.

The device for mechanochemical treatment is preferably a device using a ball, more preferably a ball mill. In order to enable production using a more-general-purpose device, a ball mill (excluding a planetary mill) is preferred. This embodiment is particularly suitable for the case where the base is an organic base.

The mechanochemical treatment may be performed with a planetary mill, but is preferably performed without a planetary mill. A planetary mill is a device that can give high energy but generates a large amount of wear debris due to the device. For example, Fig. 3 of J. Soc. Powder Technol., Japan, 44, 186-190 (2007) (https://www.jstage.jst.go.jp/article/sptj1978/44/3/44_3_186/_pdf/-char/ja) shows the relationship between the number of rotations of a planetary mill and the amount of wear debris generated due to the device; a certain amount of wear debris is generated with a planetary mill. The composition after the reaction, when used as a fluorinating agent, is preferably free from impurities. Mechanochemical treatment without a planetary mill under conditions that are less likely to generate wear debris due to a device can provide a composition suitable for a fluorinating agent. Further, the method using not a planetary mill but a general-purpose device, such as a ball mill, is advantageous in that the method can be easily adapted for industrial application.

In particular, in the case where the base is an organic base, not using a planetary mill is preferred.

The mechanochemical treatment is performed at a temperature of preferably 5°C or higher, more preferably

10°C or higher, still more preferably 15°C or higher, further preferably 20°C or higher, while preferably 300°C or lower, more preferably 250°C or lower, still more preferably 200°C or lower, further preferably 160°C or lower.

In the case where the mechanochemical treatment is performed with a ball mill (excluding a planetary ball mill), the shaking conditions can be determined in accordance with the device and ball used. For example, in a treatment using an approximately 1- to 20-mL jar and an approximately 1- to 15-mm-diameter stainless ball, the shaking can be performed under conditions including a rotational speed of preferably 100 rpm or higher, more preferably 300 rpm or higher, still more preferably 500 rpm or higher, while preferably 1800 rpm or lower, more preferably 1600 rpm or lower, still more preferably 1500 rpm or lower.

In the case where the mechanochemical treatment is performed with a ball mill (excluding a planetary ball mill), the time period of the mechanochemical treatment is preferably 5 minutes or longer, more preferably 10 minutes or longer, still more preferably 20 minutes or longer, while preferably 500 minutes or shorter, more preferably 300 minutes or shorter, still more preferably 200 minutes or shorter, further preferably 100 minutes or shorter.

The mechanochemical treatment may be performed in any atmosphere, such as in the air, in an inert gas, or in a vacuum. In order to perform the treatment at low cost, the treatment performed in the air is preferred.

The mechanochemical treatment may be performed in the absence of a solvent, and may be performed in the presence of a small amount of a solvent, if necessary. The presence of a small amount of a solvent can promote mixing of the components. The amount of the solvent used, based on the total mass of the fluorine-containing compound and the base, is preferably 0.001 μl/mg or more, more preferably 0.01 μl/mg or more, still more preferably 0.05 μl/mg or more, while preferably 3.0 μl/mg or less, more preferably 1.0 μl/mg or less, still more preferably 0.5 μl/mg or less.

The solvent for the mechanochemical treatment is preferably an organic solvent such as an ether solvent, a nitrile solvent, an ester solvent, an aromatic solvent, a hydrocarbon solvent, an alcoholic solvent, or a halogen solvent.

Examples of the organic solvent include esters such as methyl acetate, ethyl acetate, propyl acetate, n-butyl acetate, and tert-butyl acetate; ketones such as acetone, methyl ethyl ketone, and cyclohexanone; aliphatic hydrocarbons such as hexane, cyclohexane, octane, nonane, decane, undecane, dodecane, and mineral spirit; aromatic hydrocarbons such as benzene, toluene, xylene, naphthalene, and solvent naphtha; alcohols such as methanol, ethanol, tert-butanol, iso-propanol, and ethylene glycol monoalkyl ether; cyclic ethers such as tetrahydrofuran, tetrahydropyran, and dioxane; nitriles such as acetonitrile and propionitrile; dimethyl sulfoxide; amides such as N,N-dimethyl formamide and N,N-dimethyl acetamide; halogenated hydrocarbons such as dichloromethane, dichloroethane, and chloroform; and mixtures thereof.

In particular, the solvent is preferably a cyclic ether.

The mechanochemical treatment is preferably performed under dry conditions. The phrase "under dry conditions" means that the amount of liquid in the reaction system is 5% by mass or less, preferably 1% by mass or less, more preferably 0.1% by mass or less, still more preferably 0.01% by mass or less.

The mechanochemical treatment allows the fluorine-containing compound to react. This reaction may be a reaction to generate a fluoride ion, and is preferably a defluorination reaction.

The mechanochemical treatment can provide a composition containing a fluorine-containing compound, a base, and a fluoride ion (and a counterion), and this composition can directly be used as the fluorinating agent (1) of the disclosure.

The disclosure also relates to a fluorinating agent which is a composition obtained by subjecting a fluorine-containing compound and a base to mechanochemical treatment (hereinafter, also referred to as the fluorinating agent (2) of the disclosure).

The fluorinating agent (2) of the disclosure has excellent characteristics as a fluorinating agent because it is obtained by a predetermined treatment.

Examples of the fluorine-containing compound and the base in the fluorinating agent (2) of the disclosure include the same as the fluorine-containing compound and the base described for the fluorinating agent (1) of the disclosure, and the same applies to preferred embodiments thereof.

Examples of the mechanochemical treatment for the fluorinating agent (2) of the disclosure include the same treatment as the production method (2) described for the fluorinating agent (1) of the disclosure, and the same applies to preferred embodiments thereof.

Preferably, the fluorinating agent (2) of the disclosure is substantially free from a fluorine-containing organic compound. The phrase "substantially free from a fluorine-containing organic compound" means that the amount of organofluorine contained relative to the fluorinating agent (2) of the disclosure is 10% by mass or less, preferably 5% by mass or less, more preferably 3% by mass or less, still more preferably 2% by mass or less, further preferably 1% by mass or less, particularly preferably 0.1% by mass or less. The lower limit may be, but is not limited to, 0% by mass or may be 0.0001% by mass.

The amount of organofluorine in the composition is determined by combustion ion chromatography.

The organofluorine means fluorine bonded to carbon.

The fluorinating agents (1) and (2) of the disclosure each can be applied to production of a variety of compounds

having a fluorine atom, and can be particularly suitably applied to production of a fluorine-containing organic compound. The fluorinating agents (1) and (2) of the disclosure each can be suitably used as a fluorinating agent in the fluorination method of the disclosure to be described later.

The disclosure also relates to a fluorination method including fluorinating a target using a fluorinating agent that contains a fluorine-containing compound and a fluoride ion (hereinafter, also referred to as the fluorination method (1) of the disclosure).

The fluorination method (1) of the disclosure has the aforementioned structure and therefore enables fluorination using a fluorinating agent that can be produced by a simple method. The presence of a fluorine-containing compound, which absorbs less water, around a component containing a fluoride ion can reduce adhesion of this component even when water is absorbed and allows the fluorinating agent to maintain its powdery form. This therefore enables fluorination using a fluorinating agent having excellent handleability even when left in the air.

The disclosure also relates to a fluorination method including fluorinating a target using the fluorinating agent (2) of the disclosure (hereinafter, also referred to as the fluorination method (2) of the disclosure).

The fluorination method (2) of the disclosure enables a good fluorination reaction in response to the use of a fluorinating agent obtained by a predetermined treatment.

In the Description, the fluorination methods (1) and (2) of the disclosure are also collectively referred to as the "fluorination method of the disclosure".

The fluorinating agent used in the fluorination method (1) of the disclosure can be the same as the aforementioned fluorinating agent (1) of the disclosure.

The fluorinating agent used in the fluorination method (2) of the disclosure is the aforementioned fluorinating agent (2) of the disclosure.

The target to be fluorinated may be one having a group that is potentially replaced by a fluorine atom. The target is preferably a compound having at least one group that potentially nucleophilically reacts with a fluorine atom, more preferably an organic compound having at least one group that potentially nucleophilically reacts with a fluorine atom.

Examples of the group that potentially nucleophilically reacts with a fluorine atom include a chlorine atom, a bromine atom, an iodine atom, a hydrogen atom, a hydroxy group, and an organic group.

The organic group may have a carbon number of preferably 1 or greater, more preferably 2 or greater, while preferably 10 or smaller, more preferably 7 or smaller.

Examples of the organic group include an alkenyl group, an alkynyl group, $OSO_2R^2$ (wherein $R^2$ is a C1-C10 organic group), and a carboxy group.

The group that potentially nucleophilically reacts with a fluorine atom preferably includes at least one selected from the group consisting of a chlorine atom, a bromine atom, an iodine atom, and a hydroxy group, more preferably at least one selected from the group consisting of a chlorine atom, a bromine atom, and a hydroxy group, still more preferably at least one selected from the group consisting of a chlorine atom and a bromine atom, and is further preferably a chlorine atom.

The fluorination can be performed by bringing the fluorinating agent and the target into contact with each other. This contact may be achieved by any known method, although not limited thereto.

The amount of the fluorinating agent used in the fluorination relative to 1 equivalent of the group that is potentially replaced by a fluorine atom in the target is preferably 1.0 equivalents or more, more preferably 1.3 equivalents or more, still more preferably 2.0 equivalents or more, while preferably 10 equivalents or less, more preferably 5.0 equivalents or less, still more preferably 3.0 equivalents or less.

In calculation of the equivalent of the fluorinating agent, the molecular weight of the fluorinating agent is determined by the following formula:

$$\text{Molecular weight of fluorinating agent} = xM1 + M2$$

wherein M1 represents the molecular weight of the fluorine-containing compound (in the case of a polymer, its structural monomer) and M2 represents the molecular weight of the base, with the equivalent ratio of these (fluorine-containing compound:base) in the fluorinating agent being set to x:1.

The fluorination is preferably performed in the presence of a solvent. Examples of the solvent include water, an organic solvent, and a solvent mixture of these.

Examples of the organic solvent include esters such as methyl acetate, ethyl acetate, propyl acetate, n-butyl acetate, and tert-butyl acetate; ketones such as acetone, methyl ethyl ketone, and cyclohexanone; aliphatic hydrocarbons such as hexane, cyclohexane, octane, nonane, decane, undecane, dodecane, and mineral spirit; aromatic hydrocarbons such as benzene, toluene, xylene, naphthalene, and solvent naphtha; alcohols such as methanol, ethanol, tert-butanol, iso-propanol, and ethylene glycol monoalkyl ether; cyclic ethers such as tetrahydrofuran, tetrahydropyran, and dioxane; nitriles such as acetonitrile and propionitrile; dimethyl sulfoxide; amides such as N,N-dimethyl formamide and N,N-dimethyl acetamide; halogenated hydrocarbons such as dichloromethane, dichloroethane, and chloroform; and mixtures thereof.

The fluorination is performed at a temperature of preferably 5°C or higher, more preferably 10°C or higher, still more preferably 15°C or higher, further preferably 20°C or higher, while preferably 200°C or lower, more preferably 150°C or lower, still more preferably 100°C or lower, further preferably 50°C or lower.

The time period of the fluorination is preferably 5 minutes or longer, more preferably 10 minutes or longer, still more preferably 20 minutes or longer, while preferably 50 hours or shorter, more preferably 30 hours or shorter, still more preferably 20 hours or shorter, further preferably 15 hours or shorter.

The fluorination is also preferably performed under dry conditions. Using the fluorinating agent is found to promote the fluorination reaction even under dry conditions. The phrase "the fluorination is performed under dry conditions" means that the amount of liquid in the fluorination reaction system is 5% by mass or less, preferably 1% by mass or less, more preferably 0.1% by mass or less, still more preferably 0.01% by mass or less.

The fluorination is also preferably performed by mechanochemical treatment on the fluorinating agent and the target. In the case where the fluorination is performed under dry conditions, using mechanochemical treatment is particularly preferred.

The conditions for the mechanochemical treatment on the fluorinating agent and the target can be the same conditions as those described for the production method (2) for a fluorinating agent of the disclosure.

The production of the fluorinating agent and the fluorination are also preferably performed continuously. In this embodiment, preferably, both steps are performed in a single reaction vessel and the production of the fluorinating agent is directly followed by the fluorination without isolation or purification of the product. The fluorination is preferably performed by mechanochemical treatment.

The procedure of performing these steps is not specifically limited. For example, preferably, the fluorinating agent is first obtained and, without taking the contents out of the reaction vessel, the target of the fluorination is fed to the same reaction vessel, causing a fluorination reaction.

The fluorination can provide a crude product containing the fluorinated target (a compound in which a group that is potentially replaced by a fluorine atom is actually replaced by a fluorine atom).

The fluorination method of the disclosure also preferably includes purifying a crude product obtained by the fluorination and collecting the target fluorinated. The purification can be performed by any known method without limitation.

The fluorination method of the disclosure can be used to produce a variety of compounds having a fluorine atom, and can be particularly suitably used to produce a fluorine-containing organic compound.

It should be appreciated that a variety of modifications and changes in the configurations and other details may be made to the aforementioned embodiments without departing from the spirit and scope of the claims.

## EXAMPLES

The disclosure is more specifically described hereinbelow with reference to examples, but the disclosure is not limited to these examples.

<Determination of amounts of fluoride ion ($F^-$) and base contained after reaction>

These amounts were determined by ion chromatography.

The reaction product was filtered with distilled water and the liquid was removed. The residue was diluted with distilled water and subjected to determination using Tosoh IC-8100ST provided with a column (TSKgel® SuperIC-Anion HS) at an oven temperature of 40°C and a flow rate of 1.50 mL/min.

In the examples and the reference examples, the following materials (each solid at 25°C) were used.

Fluororesin A-1: PVdF (VdF homopolymer, BLD Pharmatech Ltd.), powder
Fluororesin A-2: PTFE (TFE homopolymer, Kitamura Ltd., KTL-2N)
Fluororesin A-3: PCTFE (CTFE homopolymer, Sigma-Aldrich), powder
Base B-1: tBuOK (organic base, pKa = 17)
Base B-2: KOMe (organic base, pKa = 16)
Base B-3: KOEt (organic base, pKa = 17)
Base B-4: tBuONa (organic base, pKa = 17)
Base B-5: $C_SOH \cdot H_2O$ (inorganic base, pKa = 15)
Base B-6: $Cs_2CO_3$ (inorganic base, pKa = 10)
Base B-7: KOH (inorganic base, pKa = 14.7)
Base B-8: NaOH (inorganic base, pKa = 13)

Example 1

A 10-mL stainless steel jar was charged with a stainless steel ball (10 mm), 0.5 eq. (equivalent) of Fluororesin A-1 (128 mg, 2.0 mmol, calculated based on the molecular weight (64.03) of the structural monomer VdF), 1.0 eq. of Base B-1 (449 mg, 4.0 mmol), and anhydrous THF (0.5 μL/mg fluororesin). The jar was closed and placed into a ball mill (Mixer Mill MM 400, Retsch). Mechanochemical treatment with this ball mill was performed for 60 minutes at room temperature (25°C) and 30 Hz. After the treatment was completed, the jar was opened and the product was washed with water and concentrated in a vacuum. The resulting composition (reaction mixture) was diluted with water and subjected to ion chromatography analysis.
The resulting composition was solid (powder) at 25°C and contained Fluororesin A-1, < 59% by mass of Base B-1, and 1.8% by mass of a fluoride ion.
The counterion of the fluoride ion is K. The yield of KF is shown in Table 1.
The yield was calculated with a yield of 100% indicating the case where the counterions K of Base B-1 were completely converted into KF.

Example 2

A 10-mL stainless steel jar was charged with a stainless steel ball (10 mm), 5.0 eq. (equivalent) of Fluororesin A-1 (1280 mg, 20 mmol, calculated based on the molecular weight (64.03) of the structural monomer VdF), and 1.0 eq. of Base B-1 (449 mg, 4.0 mmol). The jar was closed and placed into a ball mill (Mixer Mill MM 400, Retsch). Mechanochemical treatment with this ball mill was performed for 30 minutes at room temperature (25°C) and 30 Hz. After the treatment was completed, the jar was opened and the product was washed with water and concentrated in a vacuum. The resulting composition (reaction mixture) was diluted with water and subjected to ion chromatography analysis.
The resulting composition was solid (powder) at 25°C and contained Fluororesin A-1, Base B-1, and 2.9% by mass of a fluoride ion.
The counterion of the fluoride ion is K. The yield of KF is shown in Table 1.

Example 3

Mechanochemical treatment was performed as in Example 1 except that the amount of Fluororesin A-1 was changed to 3.0 eq.
The resulting composition was solid (powder) at 25°C and contained Fluororesin A-1, < 7.3% by mass of Base B-1, and 7.7% by mass of a fluoride ion.
The counterion of the fluoride ion is K. The yield of KF is shown in Table 1.

Example 4

Mechanochemical treatment was performed as in Example 1 except that 0.5 eq. of Fluororesin A-1 was changed to 1.0 eq. of Fluororesin A-2 (1200 mg, 12.0 mmol, calculated based on the molecular weight (100.02) of the structural monomer TFE) and that the outer surface of the reaction vessel was heated to 300°C (the internal temperature was estimated to be about 150°C) using a heat gun.
The resulting composition was solid (powder) at 25°C and contained Fluororesin A-2, Base B-1, and 4.2% by mass of a fluoride ion.
The counterion of the fluoride ion is K. The yield of KF is shown in Table 1.

Example 5

Mechanochemical treatment was performed as in Example 4 except that 1.0 eq. of Fluororesin A-2 was changed to 3.0 eq. of Fluororesin A-3, that the time period of the reaction was changed to 180 minutes, and that the outer surface of the reaction vessel was heated to 200°C (the internal temperature was estimated to be about 100°C) using a heat gun.
The resulting composition was solid (powder) at 25°C and contained Fluororesin A-3, Base B-1, and 2.0% by mass of a fluoride ion.
The counterion of the fluoride ion is K. The yield of KF is shown in Table 1.

Example 6

Mechanochemical treatment was performed as in Example 1 except that the amount of Fluororesin A-1 was changed to 2.0 eq and that the base was changed to B-2 (1.0 eq.).

The resulting composition was solid (powder) at 25°C and contained Fluororesin A-1, < 1.7% by mass of Base B-2, and 9.1% by mass of a fluoride ion.

The counterion of the fluoride ion is K. The yield of KF is shown in Table 1.

Example 7

Mechanochemical treatment was performed as in Example 6 except that the base was changed to B-3. The resulting composition was solid (powder) at 25°C, and contained Fluororesin A-1, Base B-3, and 8.8% by mass of a fluoride ion.

The counterion of the fluoride ion is K. The yield of KF is shown in Table 1.

Example 8

Mechanochemical treatment was performed as in Example 6 except that the base was changed to B-4. The resulting composition was solid (powder) at 25°C, and contained Fluororesin A-1, Base B-4, and 8.1% by mass of a fluoride ion.

The counterion of the fluoride ion is Na. The yield of NaF is shown in Table 1.

Example 9

Mechanochemical treatment was performed as in Example 6 except that the base was changed to B-5. The resulting composition was solid (powder) at 25°C, and contained Fluororesin A-1, Base B-5, and 8.0% by mass of a fluoride ion.

The counterion of the fluoride ion is Cs. The yield of CsF is shown in Table 1.

Example 10

Mechanochemical treatment was performed as in Example 6 except that the base was changed to B-6. The resulting composition was solid (powder) at 25°C, and contained Fluororesin A-1, Base B-6, and 0.2% by mass of a fluoride ion.

The counterion of the fluoride ion is Cs. The yield of CsF is shown in Table 1.

Example 11

Mechanochemical treatment was performed as in Example 1 except that the amount of Fluororesin A-1 was changed to 3.0 eq. and Base B-1 was changed to Base B-7.

The resulting composition was solid (powder) at 25°C, and contained Fluororesin A-1, Base B-7, and 5.2% by mass of a fluoride ion.

The counterion of the fluoride ion is K. The yield of KF is shown in Table 1.

Example 12

Mechanochemical treatment was performed as in Example 1 except that the amount of Fluororesin A-1 was changed to 3.0 eq. and that Base B-1 was changed to Base B-8.

The resulting composition was solid (powder) at 25°C, and contained Fluororesin A-1, Base B-8, and 1.9% by mass of a fluoride ion.

The counterion of the fluoride ion is Na. The yield of NaF is shown in Table 1.

[Table 1]

| | | Example 1 | Example 2 | Example 3 | Example 4 | Example 5 | Example 6 | Example 7 | Example 8 | Example 9 | Example 10 | Example 11 | Example 12 |
|---|---|---|---|---|---|---|---|---|---|---|---|---|---|
| Material | Fluororesin A-1 (eq.) | 0.5 | 5.0 | 3.0 | - | - | 2.0 | 2.0 | 2.0 | 2.0 | 2.0 | 3.0 | 3.0 |
| | Fluororesin A-2 (eq.) | - | - | - | 1.0 | - | - | - | - | - | - | - | - |
| | Fluororesin A-3 (eq.) | - | - | - | - | 3.0 | - | - | - | - | - | - | - |
| | Base B-1 (eq.) | 1.0 | 1.0 | 1.0 | 1.0 | 1.0 | - | - | - | - | - | - | - |
| | Base B-2 (eq.) | - | - | - | - | - | 1.0 | - | - | - | - | - | - |
| | Base B-3 (eq.) | - | - | - | - | - | - | 1.0 | - | - | - | - | - |
| | Base B-4 (eq.) | - | - | - | - | - | - | - | 1.0 | - | - | - | - |
| | Base B-5 (eq.) | - | - | - | - | - | - | - | - | 1.0 | - | - | - |
| | Base B-6 (eq.) | - | - | - | - | - | - | - | - | - | 1.0 | - | - |
| | Base B-7 (eq.) | - | - | - | - | - | - | - | - | - | - | 1.0 | - |
| | Base B-8 (eq.) | - | - | - | - | - | - | - | - | - | - | - | 1.0 |
| Condition | Reaction time period (min) | 60 | 30 | 60 | 60 | 180 | 60 | 60 | 60 | 60 | 60 | 60 | 60 |
| | Reaction temp.* (°C) | 25 | 25 | 25 | 300 (~150) | 200 (~100) | 25 | 25 | 25 | 25 | 25 | 25 | 25 |
| State | Solid or not at 25°C | Solid | Solid | Solid | Solid | Solid | Solid | Solid | Solid | Solid | Solid | Solid | Solid |
| | Form | Powder | Powder | Powder | Powder | Powder | Powder | Powder | Powder | Powder | Powder | Powder | Powder |
| Yield | Metal fluoride (%) | 14 | 66 | 97 | 47 | 49 | 95 | 98 | 85 | 82 | 2 | 69 | 24 |
| $F^-$ content | Mass% | 1.8 | 2.9 | 7.7 | 4.2 | 2.0 | 9.1 | 8.8 | 8.1 | 8.0 | 0.2 | 5.2 | 1.9 |

*In the case where heating was performed, the value refers to the heating temperature. The parenthesized value refers to the estimated internal temperature.

Example 13

An oven-dried vial provided with a magnetic stirrer was charged with nitrogen gas. Then, the composition obtained in Example 2 (172.94 mg, 0.4 mmol, 2.0 eq.), ethyl acetate (1 mL), and p-toluoyl chloride (26.43 μL, 0.2 mmol, 1.0 eq.) were successively added thereto. The contents were stirred in the vial in a nitrogen gas atmosphere at room temperature (25°C) for 12 hours. The yield of the resulting p-toluoyl fluoride was 52%, which was determined by $^{19}F$ NMR using 4-fluoroanisole as internal standard.

Reference Example 1

p-Toluoyl fluoride was obtained as in Example 4 except that the composition obtained in Example 2 was replaced with 1.5 eq. of KF obtained by spray drying. The yield was 55%.

Using the fluorinating agent of the disclosure is found to promote fluorination with an efficiency not inferior to the case of using KF, which has been conventionally known as a fluorinating agent.

Reference Example 2

The sample obtained in Example 3 was heat-dried at 120°C in advance. This sample and KF produced by spray drying were left in the air for five hours. The states after being left were then observed.

KF produced by spray drying deliquesced and solidified, i.e., failed to maintain its powdery form, which impaired the handleability in handling KF. In contrast, the sample obtained in Example 3 maintained its powdery form and maintained its handleability, so that the sample was in the form that was successfully used directly as a fluorinating agent.

The fluorinating agent of the disclosure was found to have better preservability in the air than KF, which has been conventionally known as a fluorinating agent, and to have excellent handleability.

Example 14

A glass reaction vessel provided with a magnetic stirrer was charged with the composition obtained in Example 3 (0.3 mmol, 1.5 eq.), sulfonyl chloride shown in Table 2 (R-$SO_2$Cl, 0.2 mmol, 1.0 eq.), distilled water (2.0 eq., 0.4 mmol), and acetone (0.2 M) as a solvent. The reaction product was stirred at room temperature for 30 minutes. The resulting suspension or crude product was filtered through a plug of silica eluting with EtOAc, whereby insoluble by-products were removed. The solvent was then removed by concentration under reduced pressure, whereby the corresponding sulfonyl fluoride (R-$SO_2$F) was obtained. The yields of the resulting sulfonyl fluorides are shown in Table 2 (the yields of Entry 9, Entry 10, and Entry 11 were calculated by $^{19}F$ NMR using 4-fluoroanisole as internal standard; the others were isolation yields).

[Table 2]

| Entry | R-$SO_2$Cl | R-$SO_2$F | Yield (%) | Entry | R-$SO_2$Cl | R-$SO_2$F | Yield (%) |
|---|---|---|---|---|---|---|---|
| 1 | 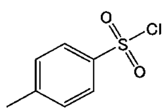 | 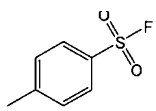 | 82 | 7 | 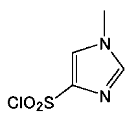 | 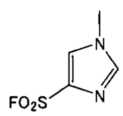 | 62 |
| 2 | 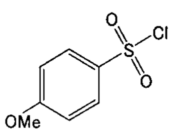 | 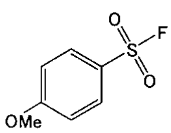 | 98 | 8 | 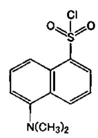 | 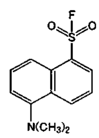 | 97 |
| 3 | 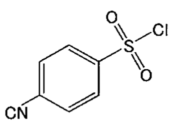 | 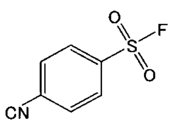 | 97 | 9 | 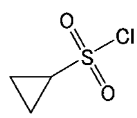 | 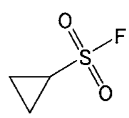 | 99 |
| 4 | 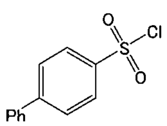 | 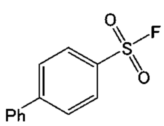 | 96 | 10 | 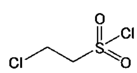 | 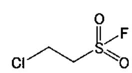 | 71 |

(continued)

| Entry | R-$SO_2$Cl | R-$SO_2$F | Yield (%) | Entry | R-$SO_2$Cl | R-$SO_2$F | Yield (%) |
|---|---|---|---|---|---|---|---|
| 5 | 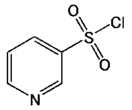 | 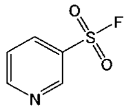 | 82 | 11 | 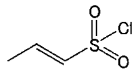 | 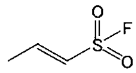 | 68 |
| 6 | 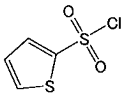 | 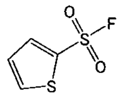 | 65 | | | | |

Example 15

An oven-dried vial provided with a magnetic stirrer was charged with nitrogen gas. Then, the composition obtained in Example 2 (0.2 mmol, 1.5 eq.), dry ethyl acetate (1 mL), and acyl chloride shown in Table 3 (R-COCl, 0.2 mmol, 1.0 eq.) were successively added thereto. The contents were stirred in the vial in a nitrogen gas atmosphere at room temperature (25°C) for 12 hours. Silica gel column chromatography was used to perform isolation and purification, whereby the corresponding acyl fluoride (R-COF) was obtained. The yields (isolation yields) of the resulting acyl fluorides are shown in Table 3.

[Table 3]

| Entry | R-COCl | R-COF | Yield (%) |
|---|---|---|---|
| 1 | 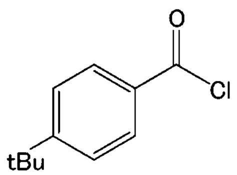 | 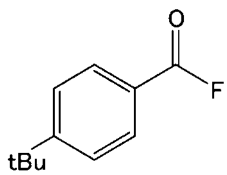 | 58 |
| 2 | 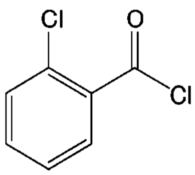 | 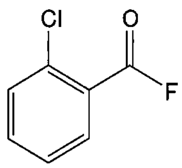 | 43 |
| 3 | 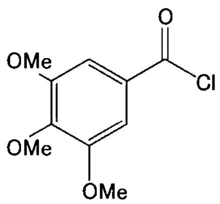 | 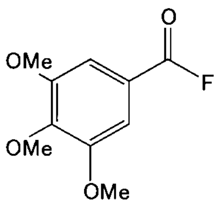 | 48 |
| 4 | 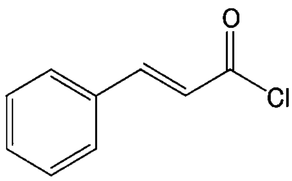 | 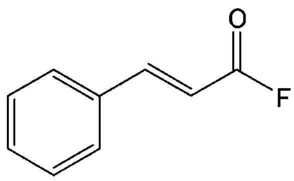 | 43 |

Example 16

A glass reaction vessel was charged with the composition obtained in Example 3 (2.0 eq., 0.4 mmol), a bromide shown in Table 4 (R-Br, 0.2 mmol, 1.0 eq.), 18-crown-6-ether (1.0 eq.), $H_2O$ (18 μL, 5.0 eq.), and anhydrous tBuOH (0.8 mL). The contents were stirred at 100°C for a time period shown in Table 4. The resulting suspension was cooled to room temperature and filtered with EtOAc, whereby insoluble by-products were removed. The solvent was then removed by concentration under reduced pressure. Silica gel column chromatography was used to perform isolation and purification,

whereby the corresponding fluoride (R-F) was obtained. The yields (isolation yields) of the resulting fluorides are shown in Table 4.

[Table 4]

| Entry | R-Br | R-F | Time (h) | Yield (%) |
|---|---|---|---|---|
| 1 | 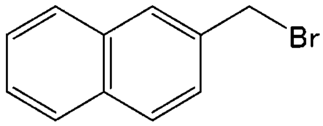 | 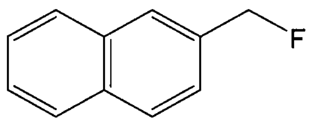 | 17 | 61 |
| 2 | 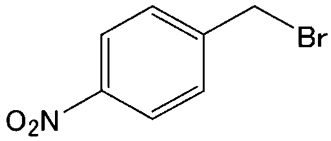 | 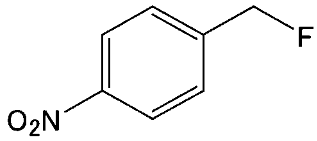 | 12 | 50 |
| 3 | 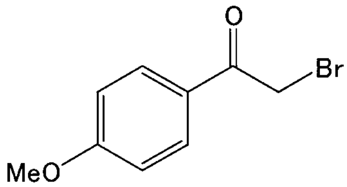 | 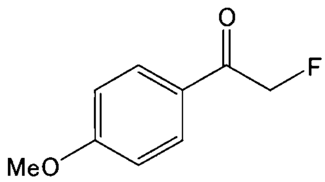 | 5 | 98 |
| 4 | 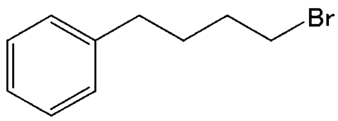 | 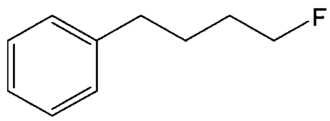 | 22 | 51 |
| 5 | 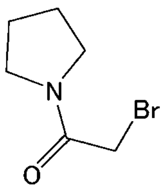 | 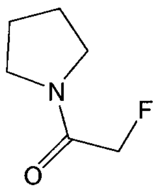 | 5 | 71 |

Example 17

A 1.5-mL stainless steel jar was charged with a stainless steel ball (5 mm), the composition obtained in Example 3 (1.5 eq., 0.3 mmol), and sulfonyl chloride shown in Table 5 ($R-SO_2Cl$, 0.2 mmol, 1.0 eq.). The jar was closed and placed into a ball mill (Mixer Mill MM 400, Retsch). Mechanochemical treatment with this ball mill was performed for five minutes at room temperature (25°C) and 30 Hz. After the treatment was completed, the jar was opened and the product was washed with ethyl acetate and concentrated in a vacuum. Thereby, the corresponding sulfonyl fluoride ($R-SO_2F$) was obtained. The yields of the resulting sulfonyl fluorides are shown in Table 5 (the yield of Entry 1 was calculated by gas chromatography (GC) using mesitylene as internal standard; the others were isolation yields).

[Table 5]

| Entry | $R-SO_2Cl$ | $R-SO_2F$ | Yield (%) |
|---|---|---|---|
| 1 | 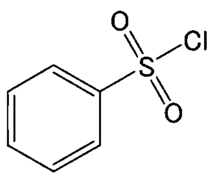 | 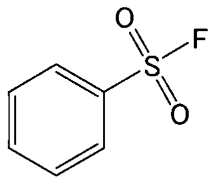 | 87 |

(continued)

| Entry | R-$SO_2$Cl | R-$SO_2$F | Yield (%) |
|---|---|---|---|
| 2 | 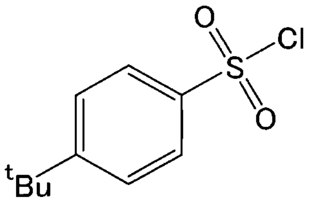 | 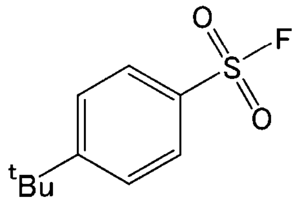 | 99 |
| 3 | 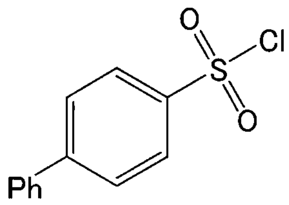 | 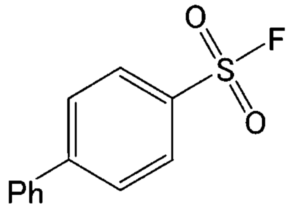 | 74 |
| 4 | 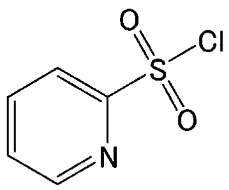 | 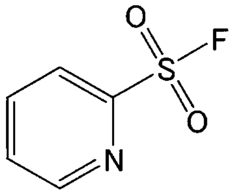 | 71 |

Example 18

A 1.5-mL stainless steel jar was charged with a stainless steel ball (5 mm), 5.0 eq. of Fluororesin A-1, and 1.1 eq. of Base B-1. The jar was closed and placed into a ball mill (Mixer Mill MM 400, Retsch). Mechanochemical treatment with this ball mill was performed for 60 minutes at room temperature (25°C) and 30 Hz. After the treatment was completed, the jar was opened and the mixture was combined with sulfonyl chloride shown in Table 6 (R-$SO_2$Cl, 0.2 mmol, 1.0 eq.). The jar was closed and mechanochemical treatment with this ball mill was performed for five minutes at room temperature (25°C) and 30 Hz. After the treatment was completed, the jar was opened and the product was washed with ethyl acetate and concentrated in a vacuum. The yields of the resulting target sulfonyl fluorides (R-$SO_2$F) were determined by $^{19}$F NMR using hexafluorobenzene as internal standard. The results are shown in Table 6.

[Table 6]

| Entry | R-$SO_2$Cl | R-$SO_2$F | Yield (%) |
|---|---|---|---|
| 1 | 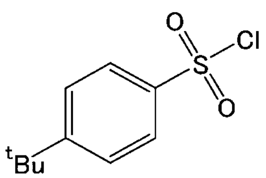 | 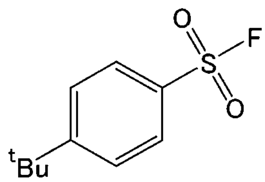 | 92 |
| 2 | 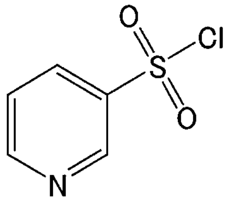 | 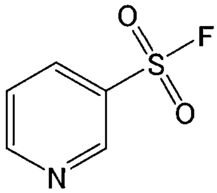 | 84 |

(continued)

| Entry | R-$SO_2Cl$ | R-$SO_2F$ | Yield (%) |
|---|---|---|---|
| 3 | 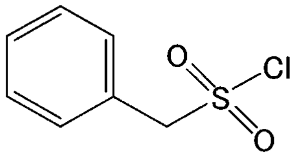 | 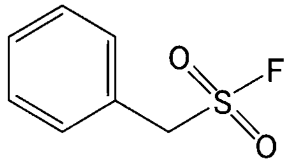 | 37 |
| 4 | 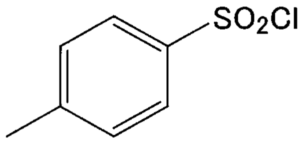 | 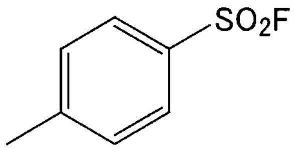 | 91 |

Example 19

A glass reaction vessel was charged with the composition obtained in Example 3 (1.5 eq., 0.3 mmol), 1-bromooctane (1.0 eq., 0.2 mmol), water (18 μL, 5.0 eq.), and anhydrous tBuOH (0.8 mL). Fluorination was performed in the same manner as in Example 13 except that the contents were stirred at 100°C for 10 h. The yield of the fluoride was 74%.

Example 20

Fluorination was performed in the same manner as in Example 13 except that 0.5 eq. of Fluororesin A-1 and 1.0 eq. of Base B-1 were used and that sulfonyl chloride was changed to p-toluenesulfonyl chloride. The fluorination yield was 1%.

Example 21

Fluorination was performed in the same manner as in Example 20 except that 2.0 eq. of Fluororesin A-1 was used. The fluorination yield was 90%.

Example 22

The composition obtained in Example 11 (1.5 eq., 0.3 mmol), p-toluenesulfonyl chloride (1.0 eq., 0.2 mmol), and water (8 μL, 2.0 eq.) were combined with each other. Fluorination was performed in the same manner as in Example 13 except for stirring at 25°C for 30 minutes. The yield of the fluoride was 58%.

Example 23

Commercially available KF (298 mg) was weighed in a glove box, stored in the air for 24 hours, and the mass (X mg) was measured. The rate of mass increase, determined by the following formula, was 20%.

$$\text{Rate of increase (\%)} = (X - 298)/298 \times 100$$

The rate of mass increase of the composition obtained in Example 1, determined in the same manner, was 12%.

## Claims

1. A fluorinating agent comprising a fluorine-containing compound and a fluoride ion.

2. The fluorinating agent according to claim 1,
wherein the fluorinating agent is solid at 25°C.

3. The fluorinating agent according to claim 1 or 2,
wherein a counterion of the fluoride ion includes at least one selected from the group consisting of an alkali metal, an

alkaline earth metal, and $NR^1_4$ (wherein $R^1$s are the same as or different from each other and are each H or a C1-C10 organic group).

**4.** The fluorinating agent according to any one of claims 1 to 3, further comprising a base.

**5.** The fluorinating agent according to claim 4, wherein the base is solid at 25°C.

**6.** The fluorinating agent according to claim 4 or 5, wherein the base has a pKa of 8 to 40.

**7.** The fluorinating agent according to any one of claims 4 to 6, wherein the base includes at least one selected from the group consisting of a compound represented by $R^{10}OM$ (wherein $R^{10}$ is H or a C1-C10 organic group; M is a metal or $NR^1_4$ (wherein $R^1$s are the same as or different from each other and are each H or a C1-C10 organic group)) and a metal carbonate.

**8.** The fluorinating agent according to any one of claims 4 to 7, wherein the base includes at least one selected from the group consisting of sodium hydroxide, potassium hydroxide, cesium hydroxide, cesium carbonate, sodium methoxide, potassium methoxide, sodium ethoxide, potassium ethoxide, sodium t-butoxide, and potassium t-butoxide.

**9.** The fluorinating agent according to any one of claims 4 to 8, wherein the base is contained in an amount of 0.1 to 70% by mass.

**10.** The fluorinating agent according to any one of claims 1 to 9, wherein the fluorine-containing compound is solid at 25°C.

**11.** The fluorinating agent according to any one of claims 1 to 10, wherein the fluorine-containing compound is a fluorine-containing polymer.

**12.** The fluorinating agent according to any one of claims 1 to 11, wherein the fluorine-containing compound is a fluorine-containing polymer containing a polymerized unit based on at least one monomer selected from the group consisting of tetrafluoroethylene, difluoroethylene, chlorotrifluoroethylene, hexafluoropropylene, perfluoro(alkyl vinyl ether), trifluoroethylene, and monofluoroethylene.

**13.** The fluorinating agent according to any one of claims 1 to 12, wherein the fluorine-containing compound includes at least one selected from the group consisting of polytetrafluoroethylene, polyvinylidene fluoride, and polychlorotrifluoroethylene.

**14.** A fluorinating agent which is a composition obtainable by subjecting a fluorine-containing compound and a base to mechanochemical treatment.

**15.** A fluorination method comprising fluorinating a target using the fluorinating agent according to claim 14.

**16.** A fluorination method comprising fluorinating a target using a fluorinating agent that comprises a fluorine-containing compound and a fluoride ion.

**17.** The fluorination method according to claim 15 or 16, wherein the fluorinating agent is solid at 25°C.

**18.** The fluorination method according to claim 16 or 17, wherein the fluorine-containing compound is solid at 25°C.

**19.** The fluorination method according to any one of claims 16 to 18, wherein the fluorine-containing compound is a fluorine-containing polymer.

**20.** The fluorination method according to any one of claims 16 to 19, wherein the fluorine-containing compound is a fluorine-containing polymer containing a polymerized unit based on at

least one monomer selected from the group consisting of tetrafluoroethylene, difluoroethylene, chlorotrifluoroethylene, hexafluoropropylene, perfluoro(alkyl vinyl ether), trifluoroethylene, and monofluoroethylene.

**21.** The fluorination method according to any one of claims 16 to 20,
wherein the fluorine-containing compound includes at least one selected from the group consisting of polytetrafluoroethylene, polyvinylidene fluoride, and polychlorotrifluoroethylene.

**22.** The fluorination method according to any one of claims 16 to 21,
wherein a counterion of the fluoride ion includes at least one selected from the group consisting of an alkali metal, an alkaline earth metal, and $NR^1_4$ (wherein $R^1$s are the same as or different from each other and are each H or a C1-C10 organic group).

**23.** The fluorination method according to any one of claims 15 to 22,
wherein the fluorinating agent further comprises a base.

**24.** The fluorination method according to claim 23,
wherein the base is solid at 25°C.

**25.** The fluorination method according to claim 23 or 24,
wherein the base includes at least one selected from the group consisting of a compound represented by $R^{10}OM$ (wherein $R^{10}$ is H or a C1-C10 organic group; M is a metal or $NR^1_4$ (wherein $R^1$s are the same as or different from each other and are each H or a C1-C10 organic group)) and a metal carbonate.

**26.** The fluorination method according to any one of claims 23 to 25,
wherein the base includes at least one selected from the group consisting of sodium hydroxide, potassium hydroxide, cesium hydroxide, cesium carbonate, sodium methoxide, potassium methoxide, sodium ethoxide, potassium ethoxide, sodium t-butoxide, and potassium t-butoxide.

**27.** The fluorination method according to any one of claims 23 to 26,
wherein the base is contained in an amount of 0.1 to 70% by mass relative to the fluorinating agent.

**28.** The fluorination method according to any one of claims 16 to 27,
wherein the fluorinating agent is obtainable by subjecting a fluorine-containing compound and a base to mechanochemical treatment.

**29.** The fluorination method according to any one of claims 15 to 28, further comprising purifying a crude product obtained by the fluorination and collecting the target fluorinated.

**30.** The fluorination method according to any one of claims 15 to 29,
wherein the fluorination is performed under dry conditions.

**31.** The fluorination method according to any one of claims 15 to 30,
wherein the fluorination is performed by subjecting the fluorinating agent and the target to mechanochemical treatment.

**32.** The fluorination method according to any one of claims 15 to 31,
wherein the production of the fluorinating agent and the fluorination are performed continuously.

**33.** The fluorination method according to any one of claims 15 to 32,
wherein the fluorinating agent is used in an amount of 1.0 to 3.0 equivalents relative to 1 equivalent of a group that is potentially replaced by a fluorine atom in the target.

**34.** The fluorination method according to any one of claims 15 to 33,
wherein the target is an organic compound containing at least one selected from the group consisting of a chlorine atom and a bromine atom.

## INTERNATIONAL SEARCH REPORT

International application No.
**PCT/JP2025/006741**

**A. CLASSIFICATION OF SUBJECT MATTER**

***C07B 39/00***(2006.01)i; ***B02C 17/14***(2006.01)i; ***B02C 19/20***(2006.01)i; ***C01D 3/02***(2006.01)i; ***C07C 17/20***(2006.01)i; ***C07C 22/08***(2006.01)i; ***C07C 49/213***(2006.01)i; ***C07C 49/217***(2006.01)i; ***C07C 51/58***(2006.01)i; ***C07C 63/70***(2006.01)i; ***C07C 303/22***(2006.01)i; ***C07C 309/80***(2006.01)i; ***C07C 309/81***(2006.01)i; ***C07C 309/85***(2006.01)i; ***C07C 309/86***(2006.01)i; ***C07C 309/87***(2006.01)i; ***C07C 309/88***(2006.01)i; ***C07D 207/08***(2006.01)i; ***C07D 213/62***(2006.01)i; ***C07D 233/84***(2006.01)i; ***C07D 333/34***(2006.01)i; ***C08F 8/26***(2006.01)i; ***C08J 3/12***(2006.01)i

FI: C07B39/00 B; C07C63/70; C07C51/58; C01D3/02; C08F8/26; C07C303/22; C07C309/86; C07C309/87; C07C309/88; C07C309/85; C07C309/80; C07C309/81; C07D207/08; C07D213/62; C07D333/34; C07D233/84; C07C17/20; C07C49/213; C07C22/08; C07C49/217; B02C17/14; B02C19/20; C08J3/12 A CEW

According to International Patent Classification (IPC) or to both national classification and IPC

**B. FIELDS SEARCHED**

Minimum documentation searched (classification system followed by classification symbols)

C07B39/00; B02C17/14; B02C19/20; C01D3/02; C07C17/20; C07C22/08; C07C49/213; C07C49/217; C07C51/58; C07C63/70; C07C303/22; C07C309/80; C07C309/81; C07C309/85; C07C309/86; C07C309/87; C07C309/88; C07D207/08; C07D213/62; C07D233/84; C07D333/34; C08F8/26; C08J3/12

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | UMEMOTO, Teruo et al., Discovery of 4-tert-Butyl-2,6-dimethylphenylsulfur Trifluoride as a Deoxofluorinating Agent with High Thermal Stability as Well as Unusual Resistance to Aqueous Hydrolysis, and Its Diverse Fluorination Capabilities Including Deoxofluoro-Arylsulfinylation with High Stereoselectivity, JOURNAL OF THE AMERICAN CHEMICAL SOCIETY, 2010, 132, 18199-18205<br>table 1, scheme 5, table 3 | 1-6, 9, 10, 16-18, 22-24, 27, 29-34 |
| A | | 7, 8, 11-15, 19-21, 25, 26, 28 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 April 2025** | **28 April 2025** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2025/006741**

**C. DOCUMENTS CONSIDERED TO BE RELEVANT**

| Category* | Citation of document, with indication, where appropriate, of the relevant passages | Relevant to claim No. |
|---|---|---|
| X | GONZALEZ-ESGUEVILLAS, Maria et al., Photoredox-catalyzed deoxyfluorination of activated alcohols with Selectfluor (Registered Trademark), TETRAHEDRON, 2023, 141, 1/7-7/7, https://doi.org/10.1016/j.tet.2023.133494<br>tables 1, 2 | 1-6, 9, 10, 16-18, 22-24, 27, 29-34 |
| A | | 7, 8, 11-15, 19-21, 25, 26, 28 |
| X | XING, Bo et al., Hypervalent Iodine(III)-Catalyzed Balz-Schiemann Fluorination under Mild Conditions, ANGEWANDTE CHEMIE INTERNATIONAL EDITION, 2018, 57, 9896-9900<br>tables 1, 2, 3 | 1-6, 9, 10, 16-18, 22-24, 27, 29-34 |
| A | | 7, 8, 11-15, 19-21, 25, 26, 28 |
| X | GOLDBERG, Nathaniel W. et al., AlkylFluor: Deoxyfluorination of Alcohols, ORGANIC LETTERS, 2016, 18, 6102-6104<br>table 1, scheme 1 | 1-6, 9, 10, 16-18, 22-24, 27, 29-34 |
| A | | 7, 8, 11-15, 19-21, 25, 26, 28 |
| X | GEARY, Gemma C. et al., Electrophilic fluorination using a hypervalent iodine reagent derived from fluoride, CHEMICAL COMMUNICATIONS, 2013, 49, 9263-9265<br>tables 1, 2 | 1-6, 9, 10, 16-18, 22-24, 27, 29-34 |
| A | | 7, 8, 11-15, 19-21, 25, 26, 28 |
| X | ILCHENKO, Nadia O. et al., Mild Silver-Mediated Geminal Difluorination of Styrenes Using an Air- and Moisture-Stable Fluoroiodane Reagent, ANGEWANDTE CHEMIE INTERNATIONAL EDITION, 2014, 53, 12897-12901<br>table 1 | 1-6, 9, 10, 16-18, 22-24, 27, 29-34 |
| A | | 7, 8, 11-15, 19-21, 25, 26, 28 |
| X | BEAULIEU, Francis et al., Aminodifluorosulfinium Tetrafluoroborate Salts as Stable and Crystalline Deoxofluorinating Reagents, ORGANIC LETTERS, 2009, 11, 5050-5053<br>table 3 | 1-6, 9, 10, 16-18, 22-24, 27, 29-34 |
| A | | 7, 8, 11-15, 19-21, 25, 26, 28 |
| X | TANG, Pingping et al., Deoxyfluorination of Phenols, JOURNAL OF THE AMERICAN CHEMICAL SOCIETY, 2011, 133, 11482-11484<br>table 1 | 1-6, 9, 10, 16-18, 22-24, 27, 29-34 |
| A | | 7, 8, 11-15, 19-21, 25, 26, 28 |
| X | AYUBA, Shinichi et al., Fluorination of Sulfides Using IF5-Et3N-3HF, BULETTIN OF THE CHEMICAL SOCIETY OF JAPAN, 2002, 75, 1597-1603<br>table 1 | 1-6, 9, 10, 16-18, 22-24, 27, 29-34 |
| A | | 7, 8, 11-15, 19-21, 25, 26, 28 |
| X | JP 2022-151785 A (DAIKIN INDUSTRIES, LTD.) 07 October 2022 (2022-10-07)<br>examples | 1-6, 9, 10, 16-18, 22-24, 27, 29-34 |
| A | | 7, 8, 11-15, 19-21, 25, 26, 28 |

**INTERNATIONAL SEARCH REPORT**
**Information on patent family members**

International application No.
**PCT/JP2025/006741**

| Patent document cited in search report | Publication date (day/month/year) | Patent family member(s) | Publication date (day/month/year) |
|---|---|---|---|
| JP 2022-151785 A | 07 October 2022 | US 2024/0018081 A1<br>examples<br>WO 2022/202888 A1<br>EP 4317124 A1<br>TW 202304843 A<br>KR 10-2023-0159876 A<br>CN 117098744 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- 2019 Nendo-ban 17019-no Kagaku Shohin (17019 Chemical Products, FY2019). The Chemical Daily Co., Ltd., 2019, 187
- *J. Soc. Powder Technol., Japan*, 2007, vol. 44, 186-190, https://www.jstage.jst.go.jp/article/sptj1978/44/3/44_3_186/_pdf/-char/ja